# EUROPEAN PATENT APPLICATION

(11) **EP 1 434 157 A2**
(43) Date of publication of application: **30.06.2004**
(21) Application number: 03255575.7
(22) Date of filing: 08.09.2003
(51) Int. Cl.: G06F 19/00

(54) **Image information processing apparatus and medical network system**

(30) Priority: 12.09.2002 JP 2002266222
(71) Applicant: KONICA CORPORATION, Tokyo 163-0512 (JP)
(72) Inventor: Onishi, Tetsuya, Tokyo 192-8505 (JP)
(74) Representative: Rees, Alexander Ellison

(57) **Abstract**

An image information processing apparatus for processing accompanying information for each image generating apparatus and output apparatus and making output apparatuses manage and use images easily. The apparatus has: an image display for displaying a medical image and accompanying information received from each medical image generating apparatus; a storage for determining and storing at least one of a first editing method for editing the accompanying information for each medical image generating apparatus and a second editing method for editing the accompanying information for each output apparatus; and an editing section comprising at least one of a first editing section for editing the accompanying information in the first editing method before displaying it on the image display and a second editing section for editing the accompanying information in the second editing method after displaying it on the image display before outputting it to each output apparatus.

## Description

### BACKGROUND OF THE INVENTION

### Field of the Invention

The invention relates to an image information processing apparatus and a medical network system capable of receiving medical images and accompanying information on the medical images from a plurality of medical image generating apparatuses and outputting the received medical images and the received accompanying information to a plurality of output apparatuses.

### Description of Related Art

An image information processing apparatus for processing a medical image such as a radiation image or the like according to an earlier development receives a medical image such as a radiation image or the like and accompanying information on the medical image from an image generating apparatus, displays the medical image and the accompanying information received, performs image processing, carries out image confirmation and outputs the medical image and the accompanying information to an external image storage apparatus, an external image forming apparatus, an external image observation apparatus or the like. The image forming apparatus forms an image on the basis of, for example, film size information of the accompanying information. When the accompanying information lacks the film size information, the image forming apparatus adds only necessary information to the accompanying information. The image storage apparatus stores the medical image with the accompanying information such as patient information, examination information or the like.

Even when the accompanying information outputted from the image generating apparatus as an input side is insufficient, the image information processing apparatus according to an earlier development cannot add necessary information to the accompanying information or it is required that a user adds necessary information to the accompanying information. Because accompanying information outputting from a plurality of image generating apparatuses is different for every apparatus, it has been difficult that the image information processing apparatus manages and uses the accompanying information in a uniform way.

When necessary information or unnecessary information for a plurality of image storage apparatuses as an output side is different, there is a case that because one image storage apparatus lacks necessary information, the image storage apparatus cannot show the most of a function thereof. As a result, construction and use of the whole system are limited. Further, there is a case that because necessary information for the image storage apparatus or the image observation apparatus in view of use or management is not outputted from the image generating apparatus, the image information processing apparatus cannot receive it.

For example, the above-described image information processing apparatus is disclosed in Japanese Patent Application Publication (Unexamined) No. Tokukai 2002-133394.

### SUMMARY OF THE INVENTION

An object of the present invention is, in view of above-described problems with an earlier development, to an image information processing apparatus and a medical network system capable of meeting each of a plurality of image generating apparatuses and each of a plurality of output apparatuses and making the plurality of output apparatuses manage and use images easily even when the plurality of image generating apparatuses output various accompanying information or do not output accompanying information or necessary information for the plurality of output apparatuses is different for every output apparatus.

In accordance with a first aspect of the present invention, an image information processing apparatus capable of receiving a medical image and accompanying information on the medical image from each of a plurality of medical image generating apparatuses, and outputting the medical image and the accompanying information to each of a plurality of output apparatuses, comprises: an image display for displaying the medical image and the accompanying information received; a storage for determining and storing at least one of a first editing method for editing the accompanying information for each of the plurality of medical image generating apparatuses and a second editing method for editing the accompanying information for each of the plurality of output apparatuses; and an editing section comprising at least one of a first editing section for editing the accompanying information outputted from each of the plurality of medical image generating apparatuses in the first editing method stored before displaying the accompanying information on the image display and a second editing section for editing the accompanying information in the second editing method stored after displaying the accompanying information on the image display before outputting the accompanying information to each of the plurality of output apparatuses.

In accordance with a second aspect of the present invention, a medical network system comprises: an image information processing apparatus; a plurality of medical image generating apparatuses each of which sends a medical image and accompanying information on the medical image to the image information processing apparatus; and a plurality of output apparatuses to each of which the image information processing apparatus outputs the medical image and the accompanying information; the image information processing apparatus comprising: an image display for displaying the medical image and the accompanying information received; a storage for determining and storing at least one of a first editing method for editing the accompanying information for each of the plurality of medical image generating apparatuses and a second editing method for editing the accompanying information for each of the plurality of output apparatuses; and an editing section comprising at least one of a first editing section for editing the accompanying information outputted from each of the plurality of medical image generating apparatuses in the first editing method stored before displaying the accompanying information on the image display and a second editing section for editing the accompanying information in the second editing method stored after displaying the accompanying information on the image display before outputting the accompanying information to each of the plurality of output apparatuses.

According to the image information processing apparatus of the first aspect or the medical network system of the second aspect of the present invention, the image information processing apparatus edits, for example, adds, changes or the like accompanying information received from each of a plurality of medical image generating apparatuses in the first editing method of the accompanying information determined and stored for each of the plurality of medical image generating apparatuses. Accordingly, it is possible to meet various accompanying information received from the plurality of medical image generating apparatuses, and to obtain necessary accompanying information even when the necessary information is not included in the accompanying information received from each of the plurality of medical image generating apparatuses. Consequently, it is possible to manage accompanying information in a uniform way. As a result, it is possible to make a plurality of output apparatuses manage and use images easily. Further, because the image information processing apparatus edits accompanying information before displaying it on the image display, and then displays edited accompanying information, it is possible that a user confirms the accompanying information. Further, the image information processing apparatus edits, for example, adds, changes or the like accompanying information received from each of the plurality of medical image generating apparatuses in the second editing method of the accompanying information determined and stored for each of the plurality of output apparatuses. Accordingly, it is possible to meet various necessary accompanying information for the plurality of output apparatuses, and to obtain necessary accompanying information for each output apparatus even when the necessary information is not included in the accompanying information received from each of the plurality of medical image generating apparatuses. Consequently, it is possible that each output apparatus shows the most of a function thereof. As a result, it is possible to make the plurality of output apparatuses manage and use images easily.

Preferably, regarding the apparatus of the first aspect of the present invention, the plurality of output apparatuses include an image storage apparatus and an image observation apparatus, or a plurality of image storage apparatuses and a plurality of image observation apparatuses.

Preferably, in the system of the second aspect of the present invention, the plurality of output apparatuses include an image storage apparatus and an image observation apparatus, or a plurality of image storage apparatuses and a plurality of image observation apparatuses.

Preferably, in the apparatus of the first aspect or the system of the second aspect of the present invention, the editing includes at least one of adding, changing, replacing, deleting, moving, copying and exchanging the accompanying information.

Preferably, in the apparatus of the first aspect or the system of the second aspect of the present invention, the accompanying information includes at least one of an image processing parameter, patient information and examination information.

Preferably, in the apparatus of the first aspect or the system of the second aspect of the present invention, an item and content of the accompanying information as an object of the editing are obtained from an external information management apparatus storing patient information, examination information and order information.

Preferably, in the apparatus of the first aspect of the present invention, communication with the plurality of medical image generating apparatuses and the plurality of output apparatuses is carried out based on a medical standard network protocol.

Preferably, in the system of the second aspect of the present invention, communication between the image information processing apparatus, the plurality of medical image generating apparatuses and the plurality of output apparatuses is carried out based on a medical standard network protocol.

Preferably, the apparatus of the first aspect of the present invention further comprises a determining section for determining whether to edit the accompanying information for each of the plurality of output apparatuses or not.

Preferably, in the system of the second aspect of the present invention, the image information processing apparatus further comprises a determining section for determining whether to edit the accompanying information for each of the plurality of output apparatuses or not.

### BRIEF DESCRIPTION OF THE DRAWINGS

The present invention will become more fully understood from the detailed description given hereinafter and the accompanying drawing given by way of illustration only, and thus are not intended as a definition of the limits of the present invention, and wherein:
FIG. 1 is a block diagram showing a structure of a medical network system according to an embodiment;
FIG. 2 is a block diagram showing a structure of a medical image information processing apparatus 50 shown in FIG. 1;
FIG. 3 is a block diagram showing a control system of the medical image information processing apparatus 50 shown in FIGS. 1 and 2;
FIGS. 4A and 4B are flowcharts showing each step of processing performed by the medical image information processing apparatus 50 shown in FIGS. 1 and 2; and
FIG. 5 is a table showing an example of patient information, examination information and image information edited uniformly in DICOM format.

### EMBODIMENTS OF THE INVENTION

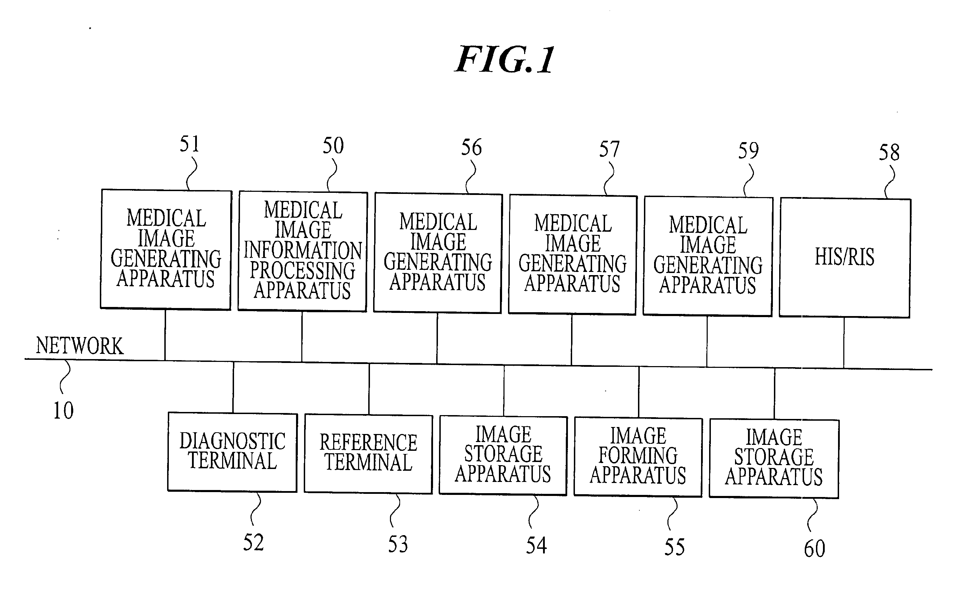

Hereinafter, a medical network system according to an embodiment of the present invention will be explained with reference to figures. FIG. 1 is a block diagram showing a structure of the medical network system according to the embodiment of the present invention.

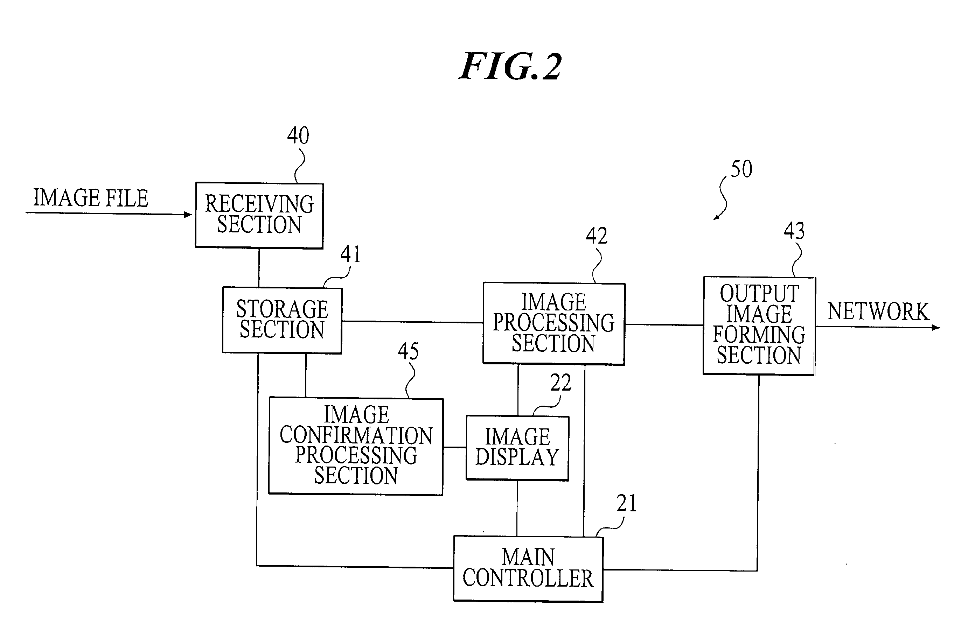

A medical image information processing apparatus 50 in FIG. 1 receives medical images so as to apply image processing on them and either outputs the image-processed medical images to an outer apparatus or displays the image-processed medical images on a screen of an image display 22 (FIG. 2), the medical image generated by scanning a photostimulable phosphor panel accumulating radiation image information of a subject (a patient) with excitation light for emitting stimulated light and photoelectrically converting the stimulated light with a photomultiplier, or the medical image generated by a modality such as a CR (Computed Radiography) or the like obtaining image information with an X-ray flat panel detector.

As shown in FIG. 1, the medical image information processing apparatus 50 constitutes the medical network system along with medical image generating apparatuses 51 and 56 for generating medical images with the above-mentioned CR, medical image generating apparatuses 57 and 59 for generating medical images with a CT (Computed Tomography), a diagnostic terminal 52 in order for a roentgenolotgist to refer to images for diagnoses, a reference terminal 53 for referring to images (not for diagnoses), a plurality of image storage apparatuses 54 and 60 capable of storing image files in an image database, and searching and reading an image from the diagnostic terminal 52, the reference terminal 53 or the like, and an image forming apparatus (an imager, a printer) 55 for outputting image information from either the medical image generating apparatus 51 or the medical image information processing apparatus 50 onto a recording medium such as film, paper or the like as a visible image.

In the medical network system in FIG. 1, each apparatus is connected on-line through a network 10 for sending and receiving information to each other. Further, an HIS (Hospital Information System) / RIS (Radiology Information System) 58 for issuing a radiographing order is connected to the network 10. For example, the medical image information processing apparatus 50 can search and obtain patient information, examination information and radiographing order information with the HIS/RIS 58.

Further, communication between the medical image information processing apparatus 50, a plurality of medical image generating apparatuses 51, 56, 57 and 59, a plurality of output apparatuses 52, 53, 54, 55 and 60, and the HIS/RIS 58 in the medical network system shown in FIG. 1 is performed based on a medical standard network protocol (for example, DICOM, HL7 or the like).

Next, the following items A - H relating to the medical image information processing apparatus 50 in FIG. 1 will be explained in detail successively.
A. Apparatus structure
B. Information
C. File
D. Input and display of main information
E. Image confirming procedures
F. Output
G. Output image formation
H. Utility function

### A. Apparatus structure

FIG. 2 is a block diagram showing an internal structure of the medical image information processing apparatus 50.
a. As shown in FIG. 2, the medical image information processing apparatus 50 comprises a main controller 21 for controlling operation of the whole apparatus, a receiving section 40 for receiving image files from the medical image generating apparatus 51 or the like, a storage section 41 that is composed of a hard disk, a RAM or the like and stores information such as the received image files or the like, an image processing section 42 for conducting image processing on image information in the image files, an output image forming section 43 for forming output images to be outputted to outer apparatuses, an image confirmation processing section 45 for making a image display 22 display reduced images for confirmation or the like of the received images thereon, and the image display 22 that is composed of a CRT display, a liquid crystal panel or the like and displays images and various types of image data such as accompanying information thereof or the like.
   The main controller 21 controls each of sections 40, 41, 42, 43, 45, the image display 22 and the like. It is possible to input various types of information to the main controller 21 or the like from an operation section 21b such as an input keyboard, a mouse or the like provided at the medical image information processing apparatus 50.
b. Functions of the medical image information processing apparatus 50 are as follows. Each function is controlled by the main controller 21.
   (1) To receive image files generated by the medical image generating apparatus 51 or the like at the receiving section 40.
   (2) To store image files in the storage section 41 temporarily.
   (3) To confirm image quality by use of reduced images prepared by the image confirmation processing section 45.
   (4) To conduct image processing at the image processing section 42.
   (5) To form output images at the output image forming section 43.
   (6) To transmit output images to outer apparatuses such as the image storage apparatus 54 or the like through the network 10.

### B. Information

Information handled by the medical image information processing apparatus 50 can be classified as follows.

### a. Condition information

The condition information is necessary for receiving image files and for outputting the received image files to outer apparatuses such as the image storage apparatus 54 or the like as processed image files. The condition information includes the following:

### (a) Image processing information

The image processing information concerns gradation processing or frequency processing at the image processing section 42.

### (b) Output apparatus information

The output apparatus information concerns outer apparatuses such as the image storage apparatus 54 or the like that reproduce and output image data. It specifies an output area, an enlargement/reduction ratio, output format (multi-format, split radiographing format), overlay, whether to conduct gradation processing and frequency processing or the like.

### (c) Overlay information

The overlay information concerns whether to overlay AP/PA, R/L, comments or the like, a position of the overlay or the like.

### (d) Information about specific designation

Information of protection: To store image files even after the image files are transmitted until protection is removed.

Information of postponement: To postpone the transmission. The information of postponement is specified when it is necessary to review and transmit the image later.

Information of priority (emergency): The information of priority is specified when preferential output such as emergency radiographing is required. In this case, the radiographing is registered at the top of a queue.

### b. Patient information

The patient information concerns a patient.

### (a) Patient ID information

The patient ID information includes an ID number, a full name, a sex, a date of birth of a patient or the like.

### (b) Order information

The order information is necessary for a doctor to request radiographing. The order information includes information concerning patient conditions, instructions of a date and a method for the requested examination or the like.

### c. Implementation information

The implementation information concerns a result of receiving and image processing.

### (a) The implementation information includes a result of receiving, a date of radiographing or the like.

### (b) A result of image processing

The result of image processing is a result of calculation of an image processing parameter. Image data is processed based on the result when being outputted.

### (c) Implementation system information

The implementation system information includes a part of system information as follows such as a system structure at the time when radiographing is conducted or the like.

### d. System information

(a) Information for managing and controlling the system in FIG. 1.
(b) A structure of the system in FIG. 1 (identification information of an outer apparatus connected thereto such as the image storage apparatus 54 or the like, a name of the apparatus or the like).
(c) A parameter or a table for controlling devices constituting the system in FIG. 1.
(d) Setting information concerning the medical image generating apparatuses 51, 56, 57 and 59 that are inputting apparatuses.
(e) Setting information concerning an output apparatus such as information of the image forming apparatus 55, HOST information or the like.

### e. Image data

(a) Image data received from the medical image generating apparatus 51.
(b) Reduced image data for display prepared from image data for image confirmation.
(c) Reduced image data for image processing of the reduced image for display at the image confirmation processing section 45.
(d) Output image data on which gradation processing, frequency processing or the like have been applied.

### C. File

A file handled by the medical image information processing apparatus 50 is stored in the storage section 41, and is classified as follows.

### a. Condition file

A condition key is a key for setting image process conditions and output conditions on image files in advance, and a condition file corresponding to each condition key is prepared. The condition file is composed of the above-mentioned condition information. The condition file is classified in terms of radiographic regions (lung, abdomen, head or the like), radiographing postures (erect posture, supine posture or the like), radiographing directions (front, side or the like), characteristics of a patient (sex, age, physique or the like), a disease name, a radiologist or the like, and a name and radiographing information corresponding to each of the above-mentioned classifying factors are set in advance. Then, the main controller 21 sets a condition file group corresponding to each of the plurality of classifications, then sets a plurality of condition files for every set condition file group, and stores the plurality of condition files in the storage section 41. Then, the optimum condition is selected at the time of receiving images.

### b. Image header file

After the image is received, an image header file is prepared. The image header file is composed of a reservation file of the radiographing (namely, radiographing information and patient informaiton) and implementation information. When a user refers to the photographing information, the patient information or the implementation information for changing, the user refers to the image header file.

### c. Reduced image file

The reduced image file is obtained by reducing image data at a certain reduction ratio.

### (a) Reduced image data for display

The reduced image data for display is used as data displayed on the image display 22 in FIG. 2.

### (b) Reduced image data for image processing

The reduced image data for image processing is used for calculating a parameter in order to conduct image processing. Its reduction ratio is determined by conforming length of one pixel after the reduction to predetermined length. As a result, after the reduction, it is possible to compensate the difference of reading pixel sizes. The calculation of a parameter for image processing is not conducted on original image data but conducted on the reduced image data for image processing.

### d. Image file

(a) An image file is composed of accompanying information (an image header) and image data.
(b) An image header is composed of the condition information, the patient information and the implementation information. When a user needs to refer to any one of the condition information, the patient information and the implementation information for changing, the user refers to the image header.

### e. Output image file

The output image file is a file of output image data on which predetermined processing among frequency processing, gradation processing, overlay processing, rotation processing and enlargement/reduction processing has been applied.

### f. System file

The system file is a file of the above-mentioned system information.

### D. Input and display of primary information

### a. Received image information display

Received images are displayed in thumbnail mode.

### b. Output information display

(1) An output size, a direction, a trimming position, an output position, an enlargement/reduction method or the like is determined and registered in the condition file in advance.
(2) When the condition key is selected, an output range and an output image range are determined under the predetermined condition and displayed on a screen of the image display 22. Here, a size of an output range display area on the screen of the image display 22 is the maximum output range at output. The output range and the output image range are graphically displayed on the output range display area so that an appropriate output range and an appropriate output image range can be selected and confirmed at each apparatus.

### c. Overlay information

(1) The overlay information specifies whether to overlay "AP", "PA", "R", "L", comments, calibration or the like, and a position of the overlay. The specification is registered in the condition file in advance.
(2) When output images are displayed within the output range display area on the screen of the image display 22, the overlay information is graphically displayed therewith.
(3) It is possible to select an appropriate overlay and specify its position.
(4) It is possible to confirm whether there is no invisible portion hidden behind the overlay. When the overlay causes inconvenience for diagnoses, the overlay can be moved.

### d. Input and output of on-line information from RIS

(1) When orders from a doctor are inputted, the inputted orders are converted into a data structure applicable to the system to be stored in the reservation file. Further, the radiographic region and the radiographing method are converted into corresponding radiographing condition keys.
(2) The image header file is converted into a data structure applicable to the RIS side to be outputted.

### e. Image list

Image files can be displayed as a list

### E. Image confirmation procedures

### a. Operation of the system at image confirmation

(1) When image files are received from the medical image generating apparatus 51, the image files are stored in the storage section 41.
(2) The image files stored in a storage medium of the storage section 41 are reduced at a predetermined reduction ratio by the image confirmation processing section 45.
(3) The reduced images are successively displayed on the screen of the image display 22.
(4) After all the reduced images are received and displayed, digital image information is image-processed with a method predetermined by the condition key to be displayed on the image display 22. The reduced images are used to determine a parameter for the image processing.
(5) When an operator observes received images displayed on the image display 22 and judges that they are normal, a key for confirming the completion of receiving is inputted from a character information inputting apparatus so as to terminate the image confirmation.
(6) When any one of the patient information, the image processing method, an output method and so on needs to be changed, it is possible to input new information from the character information inputting apparatus.
(7) When an image confirming key is inputted, the confirmation of the image is terminated and a following image is displayed automatically.
(8) When an image has a problem, it is possible to change image processing. Further, it is possible to postpone the confirmation of the image and change the detail of image processing later.
(9) When the image confirming key is inputted, the confirmation of the image is terminated and processing as follows is conducted:
   (9-1) The image file is stored in the storage section 41 as a confirmed image file.
   (9-2) Images of which confirmation has been terminated are registered in a queue for outputting to an outer apparatus. (9-3) Then, the following received image file is displayed for image confirmation.
(10) When a postponement key is inputted, image confirmation is terminated.

### F. Output

(1) Output is conducted on a non-synchronization basis with the receiving or the image confirmation.
(2) A queue is prepared and managed at each outer apparatus. The queues are operated independently, and therefore they have no influence to each other. As a result, output is done at each apparatus on a non-synchronization basis.
(3) Information on the outer apparatus having the queue in which the image is registered, is stored in the storage section 41 as a queue registration table. The queue registration table is updated and controlled for each registration and cancellation.
(4) The image registered in the queue is outputted to an outer apparatus in the order of registration thereof. The image which has been outputted is deleted from the queue.
(5) When the output is carried out, an image file stored in the storage section 41 is identified from the number registered in the queue.
(6) An output image is formed under the condition stored in the image file. The image header is converted into a data structure determined at each output apparatus, and transmitted along with image data.

### G. Forming output image

a. An output image is formed by an output image forming section 43 through the following processing:
   (1) Image data is read out of the storage section 41 to a memory for images.
   (2) Frequency processing is conducted.
   (3) Equalization processing is conducted.
   (4) Gradation processing is conducted.
   (5) Rotation of an image is carried out.
   (6) Mirror reversing is conducted.
   (7) Enlargement and reduction are carried out.
   (8) Overlay is carried out.
b. With respect to each of (2) - (8), whether it is executed or not can be specified at each output apparatus according to condition information.
c. Here, it is possible to specify that image data on which each of the specified processing of (2) - (8) has been applied is stored as a processed image data file. As a result, re-processing of common processing on the output image to each output apparatus can be avoided.
d. For example, when an enlargement ratio or a reduction ratio of an output image for an output apparatus is different depending on each output apparatus, images on which processing up to (6) has been applied may be stored. As a result, it is possible to shorten a period of time for (2) - (6) by reading the stored images to apply only (7) and (8), when the images are transmitted to another apparatus.
e. Further, processing of (5) and (6) may be conducted simultaneously with any one of (2), (3) and (4). As a result, it is possible to reduce access of memory and shorten processing time.

### H. Utility function

a. To provide a user several functions as a utility. Utility function is restricted by a password for each of a general user, a manager and a maker, respectively. In particular, for a change of information relating to images, a password of the manager is required in view of security.
b. Image file operation
   (1) An image file list, which is information concerning images stored, is displayed on the image display 22 in the order of receiving.
   (2) When a desired image is selected from the list, the patient information, the condition information and images are displayed in the same form as the screen at the image confirmation.
   (3) The patient information, the image processing method and the output method and so on can be changed.
   (4) With regard to the image specified as "postponement" at the time of radiographing, the "postponement" is removed at this point by reconfirming the image.
   (5) The order of output, and whether output to each outer apparatus is conducted can be changed.
c. Radiographing record, emission record
   (1) The radiographing information and the patient information are processed statistically for providing a user as a radiographing record and an emission record.
   (2) The number of shots on each radiographic region for a predetermined period, a list of radiographing conditions used to radiograph for a day or the like can be outputted.
d. Customizing

A screen and operating procedures can be customized to each user.

Next, the structure and the operation of the medical image information processing apparatus 50 shown in FIGS. 1 and 2 capable of meeting a plurality of medical image generating apparatuses and managing accompanying information uniformly even when the accompanying information received from the plurality of medical image generating apparatuses is different or any accompanying information is not received from the plurality of medical image generating apparatuses and of outputting necessary accompanying information for a plurality of output apparatuses even when the necessary information for the plurality of output apparatuses is different or is not prepared, will be explained with reference to figures.

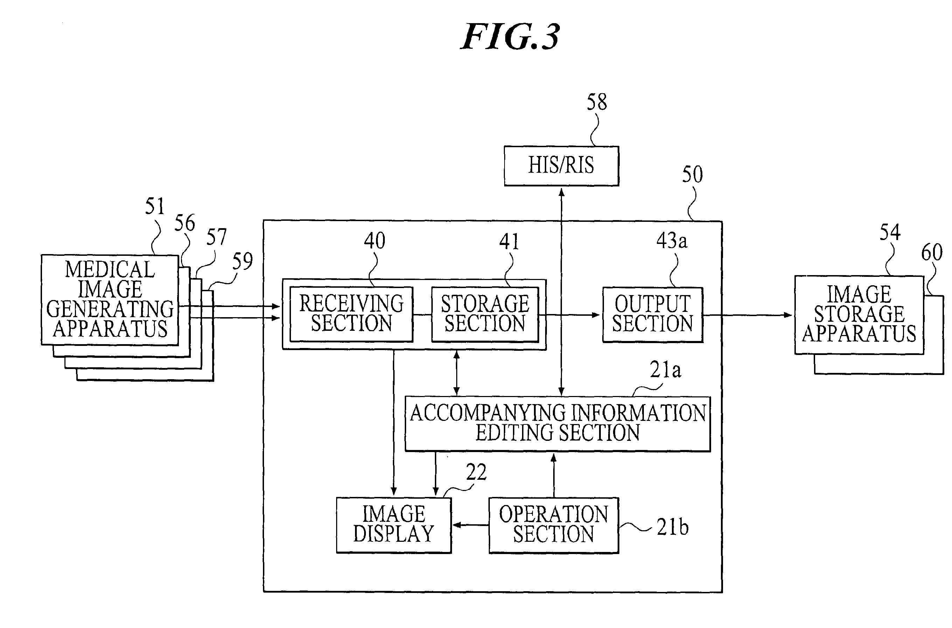

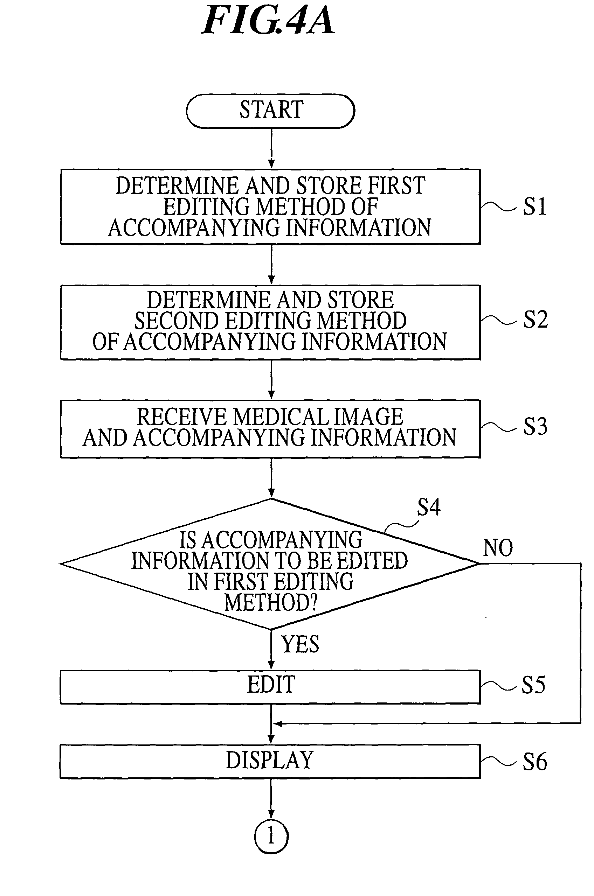

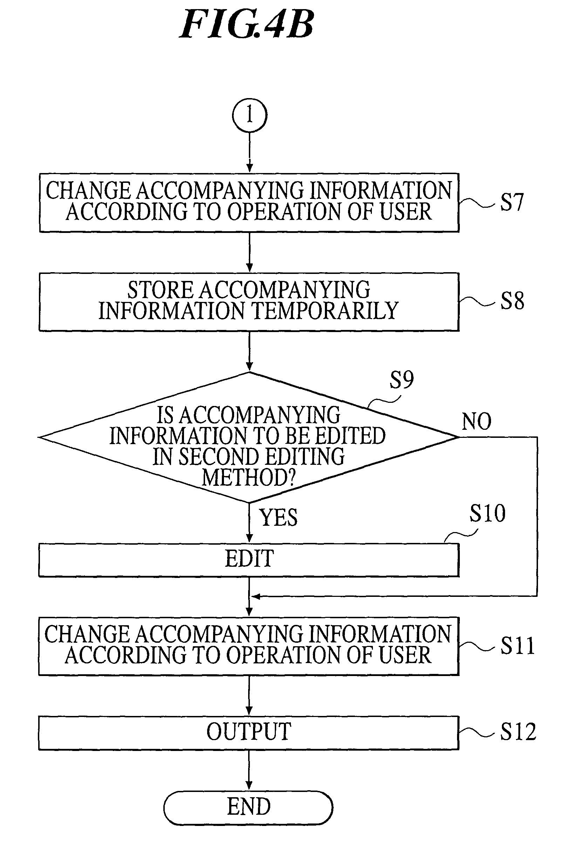

FIG. 3 is a block diagram showing a control system of the medical image information processing apparatus 50 shown in FIGS. 1 and 2. FIGS. 4A and 4B are flowcharts showing each step of processing performed by the medical image information processing apparatus 50 shown in FIGS. 1, 2 and 3.

As shown in FIG. 3, the medical image information processing apparatus 50 shown in FIGS. 1 and 2 comprises an accompanying information editing section 21a for performing predetermined editing processing to accompanying information read out of the storage section 41 which stores images received through the receiving section 40 with various types of accompanying information.

The editing processing performed by the accompanying information editing section 21a includes at least one of adding, changing, replacing, deleting, moving, copying and exchanging information. The accompanying information editing section 21a can perform predetermined editing processing. Further, a user can input information into the accompanying information editing section 21a to change, add or the like accompanying information.

The storage section 41 stores a first editing method determined so as to add, change or the like accompanying information even when the accompanying information of the plurality of medical image generating apparatuses 51, 56, 57 and 59 is different for every apparatus, and to obtain and edit necessary information even when any accompanying information is not prepared.

Further, the storage section 41 stores a second editing method determined so as to add, change or the like accompanying information even when necessary accompanying information for the plurality of image storage apparatuses 54 and 60 is different for every apparatus, and to obtain and edit necessary accompanying information for every output apparatus even when any accompanying information is not prepared.

The accompanying information editing section 21a shown in FIG. 3 automatically edits accompanying information received from each of the plurality of medical image generating apparatuses 51, 56, 57 and 59 according to the first editing method stored in the storage section 41, and further automatically edits accompanying information according to the second editing method stored in the storage section 41 before outputting the accompanying information to each of the plurality of image storage apparatuses 54 and 60.

Further, the accompanying information editing section 21a shown in FIG. 3 can obtain items and content of accompanying information from the HIS/RIS 58 when editing the accompanying information. Then, the accompanying information editing section 21a outputs the edited accompanying information and the processed image to the image storage apparatus 54 or 60 through the output section 43a of the output image forming section 43.

Next, the operation of the medical image information processing apparatus 50 shown in FIGS. 1 to 3 will be explained with reference to the flowcharts in FIGS. 4A and 4B.

First, the medical image information processing apparatus 50 determines the first editing method as content and a method for editing accompanying information of each of the plurality of medical image generating apparatuses 51, 56, 57 and 59, and stores it in the storage section 41 (S1). Further, the medical image information processing apparatus 50 determines the second editing method for editing accompanying information of each of the plurality of image storage apparatuses 54 and 60, and stores it in the storage section 41 (S2).

For example, content and a method for adding, changing, deleting or the like information of each item (or only a necessary item) of the accompanying information may be determined as the editing method. The item of the accompanying information includes, for example, a patient ID, Study instance UID or the like. Therefore, the medical image information processing apparatus 50 may add a name mentioned in Chinese characters to a patient name, or change SOP instance UID.

Next, after the medical image information processing apparatus 50 receives a medical image and accompanying information on the medical image, for example, from the medical image generating apparatus 51 (S3), when the accompanying information is to be edited in the first editing method determined for the medical image generating apparatus 51 (S4; YES), the medical image information processing apparatus 50 edits, for example, adds, changes, deletes or the like the accompanying information in the first editing method (S5), and displays the received medical image and the edited accompanying information on a screen of the image display 22 (S6). On the other hand, when the accompanying information is not to be edited in the first editing method (S4; NO), the medical image information processing apparatus 50 displays the medical image and the accompanying information received on the screen of the image display 22 (S6).

Then, when the displayed accompanying information is necessary to be changed, the medical image information processing apparatus 50 changes the accompanying information according to operation of the user through the operation section 21b (S7), and stores the accompanying information changed according to the operation of the user (or the accompanying information which has been not changed) in the storage section 41 temporarily (S8).

Then, when the accompanying information is to be edited in the second editing method determined, for example, for the image storage apparatus 54 as the output apparatus (S9; YES), the medical image information processing apparatus 50 edits, for example, adds, changes, deletes or the like the accompanying information in the second editing method (S10). Because the medical image and the accompanying information are displayed on the screen, when the displayed accompanying information is necessary to be changed or the like, the medical image information processing apparatus 50 changes the accompanying information according to operation of the user through the operation section 21b (S11), and outputs the medical image and the accompanying information changed according to the operation of the user (or the accompanying information which has been not changed) to the image storage apparatus 54 (S12).

On the other hand, when the accompanying information is not to be edited in the second editing method (S9; NO), the medical image information processing apparatus 50 changes the accompanying information according to operation of the user through the operation section 21b when it is necessary (S11), and outputs the medical image and the accompanying information to the image storage apparatus 54 (S12).

According to the processing, the medical image information processing apparatus 50 edits the accompanying information for the medical image generating apparatus 51. However, when receiving a medical image and accompanying information from any of other medical image generating apparatuses 56, 57 and 59, the medical image information processing apparatus 50 edits the accompanying information in the editing method suitable for the medical image generating apparatus like the medical image generating apparatus 51. Further, although the medical image information processing apparatus 50 edits the accompanying information for the image storage apparatus 54, the medical image information processing apparatus 50 edits the accompanying information in the editing method suitable for the image storage apparatus 60 as the output apparatus like the image storage apparatus 54.

As described above, according to the embodiment shown in FIGS. 4A and 4B, even when accompanying information outputted from a plurality of medical image generating apparatuses is different for every apparatus or any accompanying information is not prepared, the medical image information processing apparatus 50 adds or changes accompanying information to be uniform. Consequently, it is possible to provide sufficient accompanying information. As a result, it is possible that the image storage apparatuses 54 and 60 manage and use images easily based on the accompanying information.

Further, even when the format or the meaning of the item of accompanying information is different for every medical image generating apparatus, because the medical image information processing apparatus 50 uniforms the format or the meaning of the item among the medical image generating apparatuses, it is possible to display the accompanying information for the user easily, or to make the user operate the accompanying information easily. Further, because the medical image information processing apparatus 50 edits accompanying information to be outputted for every image storage apparatus as the output side, it is possible that each image storage apparatus shows the most of a function thereof. As a result, it is possible to broaden the construction or the use of the system.

Further, because it is unnecessary for the user to add necessary accompanying information even when necessary, it is possible to use the system easily. Further, because accompanying information is edited when the medical image and the accompanying information are received or outputted, it is possible to edit the accompanying information easily without affecting the whole system when another apparatus is added to the system or the system is extended and connected to another system.

In FIGS. 4A and 4B, the medical image information processing apparatus 50 determines whether to edit accompanying information in the first editing method after receiving the medical image and the accompanying information before displaying them on the screen (S4). However, Steps S4 and S5 may be omitted, and the medical image information processing apparatus 50 may edit the accompanying information only in the second editing method after displaying the medical image and the accompanying information before outputting them (S9 and S10). By contraries, Steps S9 and S10 may be omitted, and the medical image information processing apparatus 50 may edit the accompanying information only in the first editing method.

Further, the medical image information processing apparatus 50 can determine whether to edit, for example, add, change, delete or the like accompanying information for every medical image generating apparatus or for every output apparatus according to operation of the user through the operation section 21b or the like, in above-described Step S4 or S9, respectively. Furthermore, the medical image information processing apparatus 50 may determine whether to add, change, delete or the like information for every item of the accompanying information.

Further, when editing as described above (S5 or S10), the medical image information processing apparatus 50 may obtain the items and the content of the accompanying information as an object of the accompanying information editing section 21a shown in FIG. 3, from the HIS/RIS 58.

Next, a specific example of the item on the accompanying information will be given as follows. In each editing method, the content and the method for editing, for example, adding, changing, deleting or the like the accompanying information are determined for every item or for every necessary item.
(1) Examination information (an apparatus type, order, a region, a consulting department, a radiographing method, the date or the like)
(2) Examination, each of a series of IDs, instance UID, an accession number, reference/generator SOP instance UID
(3) Input CH
(4) Patient information (a patient ID, a patient name, a sex, patient's birthday, height, weight or the like)
(5) Radiographing condition (a plate position, irradiation amount, sensitivity or the like)
(6) Radiographing apparatus information (a name, a version, the adjustment date or the like)

Next, a specific example of the content and the method for editing the accompanying information in each editing method will be explained as follows.
(1) When a Study ID is not included in accompanying information on received data, content of a patient ID item is copied to a study ID item. When the patient ID item is not prepared, a new Study ID is prepared and written in the study ID item.
(2) When any item corresponding to accompanying information on an inpatient or an outpatient included in received data is not prepared for the output apparatus side, the accompanying information on the inpatient or the outpatient is moved to an item for a patient's state, a present patient's location and a patient's address in hospital.
(3) When any item corresponding to accompanying information on the inpatient or the outpatient included in received data is not prepared for the output apparatus side, "inpatient" or "outpatient" is added to the top or last of content of the item for the patient's state, the present patient's location and the patient's address in hospital.
(4) When a radiographing pattern name according to a radiographic region proper to the apparatus is not included in received data, the radiographing pattern name is overwritten in an item for processing description of the processing apparatus.
(5) When only a patient name mentioned in the Latin alphabet is included in a patient name, a patient name mentioned in Chinese characters is searched based on the patient ID or the patient name mentioned in the Latin alphabet on the MWL (Modality Work List) of DICOM of the HIS/RIS, and added to the item for the patient name. Besides, there is a case that patient information, for example, patient's birthday, height, weight or the like is added to the item for the patient name.
(6) When an accession number, a Study ID, Study instance UID, a series ID and series UID are included in the received image, and are serial numbers for controlling the image generating apparatus, each number is searched based on the patient ID or the name in the HIS/RIS and overwritten in the item.
(7) When examinations based on Study instance UIDs of images outputted from image generating apparatuses A and B are recognized as the same examination because the Study instance UIDs are the same as each other, although the image number 1 has been attached to each of the images, consecutive image numbers 1 and 2 are newly attached to the images, respectively.
(8) When the Study ID is included but Study instance UID is not included as a result of search in the HIS/RIS, new Study instance UID is prepared based on the Study ID. More specifically, new Study instance UID is prepared based on at least the apparatus type number, the examination date and the Study ID.
(9) When SOP instance UIDs attached to images respectively are outputted to each of a plurality of image storage apparatuses, the SOP instance UIDs are unified into one SOP instance UID.
(10) On the contrary to the above-described (9), when SOP instance UID is outputted to each of a plurality of image storage apparatuses, the SOP instance UID is changed according to each of the plurality of image storage apparatuses. Further, the user may change the SOP instance UID even when outputting it to the image storage apparatus.
(11) When an overlay is not displayed on an image storage apparatus, information on the overlay is deleted without being sent to the image storage apparatus. Further the overlay is inserted in the image (the image itself is changed), and information on the overlay is not sent to the image storage apparatus. The overlay includes at least one of the patient name, an arrow, a text described by the user and scale display at intervals on the image.
(12) The radiographic region which is mentioned in English (in case of DICOM) in communication between apparatuses is replaced to one mentioned in Japanese.
(13) SOP instance UID of the received original image is copied to SOP instance UID of the reference or generator image.
(14) The modality obtained from the HIS/RIS based on the MWL is moved to the accompanying information.
(15) Content of a patient comment and content of an examination comment are exchanged for each other.

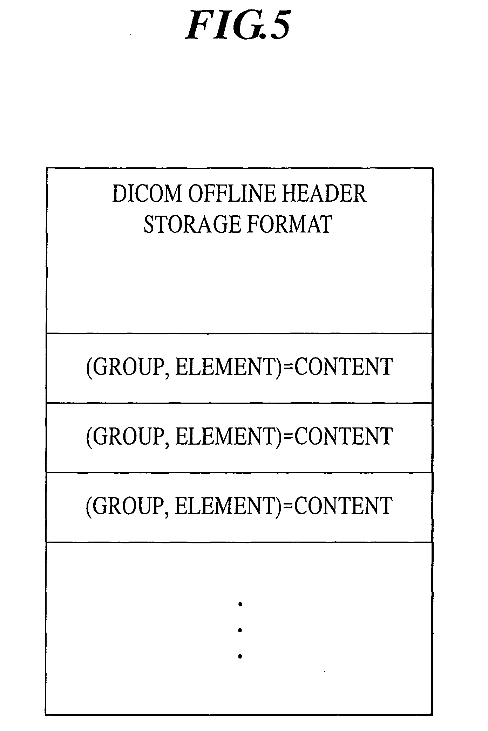

FIG. 5 shows an example of editing patient information, examination information and image information in DICOM format in a uniform way. (GROUP, ELEMENT) in FIG. 5 is a unique number showing each item of the patient information, the examination information and the image information. For example, (0010,0010) shows the patient name. As shown in FIG. 5, for example, it is possible to describe each information in the format in a uniform way.

A program programmed so as to execute each step in the above-described flowcharts shown in FIGS. 4A and 4B is stored in the storage section 41 of the medical image information processing apparatus 50 shown in FIGS. 2 and 3. Therefore, the main controller 21 can read the program out of the storage section 41 and execute the program as necessary.

Although the present invention has been explained according to the above-described embodiment, it should also be understood that the present invention is not limited to the embodiment and various changed and modifications may be made to the invention without departing from the gist thereof.

For example, each of the medical image generating apparatuses 51, 56, 57 and 59 may be a radiographing modality other than the CR or the CT as described above. For example, it is needless to say that each medical image generating apparatus may be a radiographing modality of medical image generating apparatus such as a MRI (magnetic resonance imaging), a DR (digital radiography), an US (ultrasound) or the like. Further, the radiographing modality of medical image generating apparatus may be connected to the medical network system of the present invention.

Further, although the present invention has been explained in case that the output apparatus is the image storage apparatus as an example in FIGS. 4A and 4B, the present invention is not limited to it. It is needless to say that the output apparatus may be an image observation apparatus such as the diagnostic terminal 52 or the reference terminal 53 shown in FIG. 1.

According to the present invention, it is possible to meet each image generating apparatus or each output apparatus even when accompanying information outputted from a plurality of image generating apparatuses is different for every apparatus, no accompanying information is outputted from any one of the plurality of image generating apparatuses, or necessary information for a plurality of output apparatuses is various. As a result, it is possible to provide the image information processing apparatus and the medical network system capable of making the plurality of output apparatuses manage and use images easily.

The entire disclosure of Japanese Patent Application No. Tokugan 2002-266222 filed on September 12, 2002 including specification, claims, drawings and summary are incorporated herein by reference in its entirety.

## Claims

1. An image information processing apparatus capable of receiving a medical image and accompanying information on the medical image from each of a plurality of medical image generating apparatuses, and outputting the medical image and the accompanying information to each of a plurality of output apparatuses, the apparatus comprising:
an image display for displaying the medical image and the accompanying information received;
a storage for determining and storing at least one of a first editing method for editing the accompanying information for each of the plurality of medical image generating apparatuses and a second editing method for editing the accompanying information for each of the plurality of output apparatuses; and
an editing section comprising at least one of a first editing section for editing the accompanying information outputted from each of the plurality of medical image generating apparatuses in the first editing method stored before displaying the accompanying information on the image display and a second editing section for editing the accompanying information in the second editing method stored after displaying the accompanying information on the image display before outputting the accompanying information to each of the plurality of output apparatuses.

2. The apparatus of claim 1, wherein the plurality of output apparatuses include an image storage apparatus and an image observation apparatus.

3. The apparatus of any one of claims 1 and 2, wherein the plurality of output apparatuses include a plurality of image storage apparatuses and a plurality of image observation apparatuses.

4. The apparatus of any one of claims 1 to 3, wherein the editing includes at least one of adding, changing, replacing, deleting, moving, copying and exchanging the accompanying information.

5. The apparatus of any one of claims 1 to 4, wherein the accompanying information includes at least one of an image processing parameter, patient information and examination information.

6. The apparatus of any one of claims 1 to 5, wherein an item and content of the accompanying information as an object of the editing are obtained from an external information management apparatus storing patient information, examination information and order information.

7. The apparatus of any one of claims 1 to 6, wherein communication with the plurality of medical image generating apparatuses and the plurality of output apparatuses is carried out based on a medical standard network protocol.

8. The apparatus of any one of claims 1 to 7, further comprising a determining section for determining whether to edit the accompanying information for each of the plurality of output apparatuses or not.

9. A medical network system comprising:
an image information processing apparatus;
a plurality of medical image generating apparatuses each of which sends a medical image and accompanying information on the medical image to the image information processing apparatus; and
a plurality of output apparatuses to each of which the image information processing apparatus outputs the medical image and the accompanying information;
the image information processing apparatus comprising:
an image display for displaying the medical image and the accompanying information received;
a storage for determining and storing at least one of a first editing method for editing the accompanying information for each of the plurality of medical image generating apparatuses and a second editing method for editing the accompanying information for each of the plurality of output apparatuses; and
an editing section comprising at least one of a first editing section for editing the accompanying information outputted from each of the plurality of medical image generating apparatuses in the first editing method stored before displaying the accompanying information on the image display and a second editing section for editing the accompanying information in the second editing method stored after displaying the accompanying information on the image display before outputting the accompanying information to each of the plurality of output apparatuses.

10. The system of claim 9, wherein the plurality of output apparatuses include an image storage apparatus and an image observation apparatus.

11. The system of any one of claims 9 and 10, wherein the plurality of output apparatuses include a plurality of image storage apparatuses and a plurality of image observation apparatuses.

12. The system of any one of claims 9 to 11, wherein the editing includes at least one of adding, changing, replacing, deleting, moving, copying and exchanging the accompanying information.

13. The system of any one of claims 9 to 12, wherein the accompanying information includes at least one of an image processing parameter, patient information and examination information.

14. The system of any one of claims 9 to 13, wherein an item and content of the accompanying information as an object of the editing are obtained from an external information management apparatus storing patient information, examination information and order information.

15. The system of any one of claims 9 to 14, wherein communication between the image information processing apparatus, the plurality of medical image generating apparatuses and the plurality of output apparatuses is carried out based on a medical standard network protocol.

16. The system of any one of claims 9 to 15, wherein the image information processing apparatus further comprises a determining section for determining whether to edit the accompanying information for each of the plurality of output apparatuses or not.
